# EUROPEAN PATENT APPLICATION

(11) **EP 4 696 709 A1**
(43) Date of publication of application: **18.02.2026**
(21) Application number: 25160905.3
(22) Date of filing: 28.02.2025
(51) Int. Cl.: C07K 14/715, A61K 38/00

(54) **VARIANTS OF ETANERCEPT AND RELATED PROCESSES**

(30) Priority: 06.12.2024 CN 202411793225; 13.01.2025 CN 202510051620
(71) Applicant: QILU PHARMACEUTICAL CO., LTD., Jinan, Shandong 250100 (CN)
(72) Inventor: SUN, Bo, Jinan, Shandong, 250100 (CN); JIA, Guodong, Jinan, Shandong, 250100 (CN); AN, Zhenming, Jinan, Shandong, 250100 (CN); LIN, Ming, Jinan, Shandong, 250100 (CN); WEI, Songhuan, Jinan, Shandong, 250100 (CN); ZHANG, Shouyu, Jinan, Shandong, 250100 (CN); XING, Tingting, Jinan, Shandong, 250100 (CN); LI, Hai, Jinan, Shandong, 250100 (CN); ZHU, Yanfeng, Jinan, Shandong, 250100 (CN)
(74) Representative: Bandpay & Greuter

(57) **Abstract**

The present disclosure relates to variants of etanercept and related processes. The variants are specific truncation variants of etanercept. Also provided herein are a pharmaceutical formulation comprising the variants. Additonally, provided herein is a method for decreasing the variants. The method can effectively optimize the amount of the truncation variants, with simplicity in process and ease in operation.

## Description

### RELATED APPLICATION

The present application claims priority to Chinese Patent Application No. 202411793225.7, filed on December 6, 2024, and Chinese Patent Application No. 202510051620.6, filed on January 13, 2025, the entire contents of which are incorporated herein by reference in their entirety for all purposes.

### TECHNICAL FIELD

The present disclosure relates to the field of pharmaceuticals, and more particularly to variants of etanercept and related processes.

### BACKGROUND

Tumor necrosis factor (TNF) is a key pathogenic factor leading to rheumatoid arthritis. Etanercept is a recombinant protein of human type II tumor necrosis factor receptor, consisting of an extracellular ligand binding portion of the type II tumor necrosis factor receptor and an Fc fragment of human IgG1. Etanercept can bind free TNF with high affinity and becomes a natural antagonist of TNF by preventing the binding of a TNF molecule to its cellular receptor. It blocks TNF-mediated cellular responses by inhibiting the biological activity of TNF, thereby fundamentally blocking disease progression. It is also involved in modulation of biological responses controlled by other downstream molecules (e.g., cytokines, adhesion molecules, or proteases) induced or regulated by TNF. Etanercept can be used to relive common symptoms such as joint swelling and pain, joint stiffness and morning stiffness, and prevent joint injuries and improve joint function. It has biological activies of neutralizing TNF-α and TNF-β.

With the continuous development and progress in biotechnology and bioengineering, and along with the maturation and ongoing improvement of upstream cell culture technologies, the protein expression levels have been increasing. The contents of related substances of the target proteins such as variants or impurities become more significant. Further investigations are required for the analysis of variants or impurities in etanercept formulations and processes for reducing the amounts of the variants or impurities.

### SUMMARY OF THE INVENTION

The present disclosure relates to specific variants of etanercept and related processes for reducing amounts of the variants.

In an aspect, the present disclosure provides a composition comprising etanercept and a truncation variant thereof, said truncation variant comprising one or more of:
(1) a truncation variant resulting from a breakage between positions G234/D235 in at least one chain of etanercept,
(2) a truncation variant resulting from a breakage between positions D235/E236 in at least one chain of etanercept,
(3) a truncation variant resulting from a breakage between positions T233/G234 in at least one chain of etanercept,
(4) a truncation variant resulting from a breakage between positions S239/C240 in at least one chain of etanercept,
(5) a truncation variant resulting from a breakage between positions K238/S239 in at least one chain of etanercept, and
(6) a truncation variant resulting from a breakage between positions C240/D241 in at least one chain of etanercept.

The above positions are numbered with reference to SEQ ID NO: 1.

Etanercept is formed by two identical peptide chains linked by disulfide bonds. In some embodiments, the sequence of each of the two identical peptide chains of etanercept is set forth in SEQ ID NO: 1.

In preferable embodiments, the truncation variants comprise one or more of:
(1) a truncation variant resulting from a breakage between positions G234/ D235 in one chain of etanercept and one of the following options a to g for the other chain:
   a. no breakage occurs, and the sequence is as shown in SEQ ID NO: 1;
   b. a breakage occurs between positions G234/D235;
   c. a breakage occurs between positions D235/E236;
   d. a breakage occurs between positions T233/ G234;
   e. a breakage occurs between positions S239/C240;
   f. a breakage occurs between positions K238/S239;
   g. a breakage occurs between positions C240/D241;
(2) a truncation variant resulting from a breakage between positions D235/E236 in one chain of etanercept and one of the following options a to g for the other chain:
   a. no breakage occurs, and the sequence is as shown in SEQ ID NO: 1;
   b. a breakage occurs between positions G234/D235;
   c. a breakage occurs between positions D235/E236;
   d. a breakage occurs between positions T233/ G234;
   e. a breakage occurs between positions S239/C240;
   f. a breakage occurs between positions K238/S239;
   g. a breakage occurs between positions C240/D241;
(3) a truncation variant resulting from a breakage between positions T233/G234 in one chain of etanercept and one of the following options a to g for the other chain:
   a. no breakage occurs, and the sequence is as shown in SEQ ID NO: 1;
   b. a breakage occurs between positions G234/D235;
   c. a breakage occurs between positions D235/E236;
   d. a breakage occurs between positions T233/ G234;
   e. a breakage occurs between positions S239/C240;
   f. a breakage occurs between positions K238/S239;
   g. a breakage occurs between positions C240/D241;
(4) a truncation variant resulting from a breakage between positions S239/C240 in one chain of etanercept and one of the following options a to g for the other chain:
   a. no breakage occurs, and the sequence is as shown in SEQ ID NO: 1;
   b. a breakage occurs between positions G234/D235;
   c. a breakage occurs between positions D235/E236;
   d. a breakage occurs between positions T233/ G234;
   e. a breakage occurs between positions S239/C240;
   f. a breakage occurs between positions K238/S239;
   g. a breakage occurs between positions C240/D241;
(5) a truncation variant resulting from a breakage between positions K238/S239 in one chain of etanercept and one of the following options a to g for the other chain:
   a. no breakage occurs, and the sequence is as shown in SEQ ID NO: 1;
   b. a breakage occurs between positions G234/D235;
   c. a breakage occurs between positions D235/E236;
   d. a breakage occurs between positions T233/ G234;
   e. a breakage occurs between positions S239/C240;
   f. a breakage occurs between positions K238/S239;
   g. a breakage occurs between positions C240/D241; and
(6) a truncation variant resulting from a breakage between positions C240/D241 in one chain of etanercept and one of the following options a to g for the other chain:
   a. no breakage occurs, and the sequence is as shown in SEQ ID NO: 1;
   b. a breakage occurs between positions G234/D235;
   c. a breakage occurs between positions D235/E236;
   d. a breakage occurs between positions T233/ G234;
   e. a breakage occurs between positions S239/C240;
   f. a breakage occurs between positions K238/S239;
   g. a breakage occurs between positions C240/D241.

The above positions are numbered with reference to SEQ ID NO: 1.

In some embodiments, the truncation variant is in a total amount of less than or equal to about 5% in the composition, as determined by a SEC-HPLC method.

In preferable embodiments, the truncation variant is in a total amount of less than or equal to about 4.5%, less than or equal to about 4%, less than or equal to about 3.5%, less than or equal to about 3%, less than or equal to about 2.5%, less than or equal to about 2%, less than or equal to about 1.7%, or less than or equal to about 1.5% in the composition.

In preferable embodiments, the truncation variant is in a total amount of greater than or equal to about 0.01%, greater than or equal to about 0.05%, greater than or equal to about 0.1%, greater than or equal to about 0.5%, or greater than or equal to about 1% in the composition.

In some embodiments, the composition is prepared by a method comprising a step of purifying a cell culture harvest solution comprising etanercept protein molecules by protein A affinity chromatography, wherein sodium sulfate is added to an elution buffer for the protein A affinity chromatography used in the step of purification.

In another aspect, the present disclosure provides a pharmaceutical formulation comprising a composition described in the present disclosure, and one or more pharmaceutically acceptable carriers.

In another aspect, the present disclosure provides a method for purifying etanercept, comprising purifying a composition containing etanercept protein molecules by protein A affinity chromatography, wherein sodium sulfate is added to an elution buffer for the protein A affinity chromatography used in the step of purification.

In some embodiments, the composition containing etanercept protein molecules is a cell culture harvest solution.

In another aspect, the present disclosure provides a method for reducing the amount of a truncation variant of etanercept in an etanercept-containing composition, comprising purifying an etanercept-containing composition, e.g. a cell culture harvest solution, by protein A affinity chromatography, wherein sodium sulfate is added to an elution buffer for the protein A affinity chromatography used in the step of purification, wherein the truncation variant comprises one or more of:
(1) a truncation variant resulting from a breakage between positions G234/D235 in at least one chain of etanercept,
(2) a truncation variant resulting from a breakage between positions D235/E236 in at least one chain of etanercept,
(3) a truncation variant resulting from a breakage between positions T233/G234 in at least one chain of etanercept,
(4) a truncation variant resulting from a breakage between positions S239/C240 in at least one chain of etanercept,
(5) a truncation variant resulting from a breakage between positions K238/S239 in at least one chain of etanercept, and
(6) a truncation variant resulting from a breakage between positions C240/D241 in at least one chain of etanercept.

The above positions are numbered with reference to SEQ ID NO: 1.

In some embodiments, the sequence of each of the two identical peptide chains of etanercept is set forth in SEQ ID NO: 1.

In some preferable embodiments, the truncation variants comprise one or more of:
(1) a truncation variant resulting from a breakage between positions G234/ D235 in one chain of etanercept and one of the following options a to g for the other chain:
   a. no breakage occurs, and the sequence is as shown in SEQ ID NO: 1;
   b. a breakage occurs between positions G234/D235;
   c. a breakage occurs between positions D235/E236;
   d. a breakage occurs between positions T233/ G234;
   e. a breakage occurs between positions S239/C240;
   f. a breakage occurs between positions K238/S239;
   g. a breakage occurs between positions C240/D241;
(2) a truncation variant resulting from a breakage between positions D235/E236 in one chain of etanercept and one of the following options a to g for the other chain:
   a. no breakage occurs, and the sequence is as shown in SEQ ID NO: 1;
   b. a breakage occurs between positions G234/D235;
   c. a breakage occurs between positions D235/E236;
   d. a breakage occurs between positions T233/ G234;
   e. a breakage occurs between positions S239/C240;
   f. a breakage occurs between positions K238/S239;
   g. a breakage occurs between positions C240/D241;
(3) a truncation variant resulting from a breakage between positions T233/G234 in one chain of etanercept and one of the following options a to g for the other chain:
   a. no breakage occurs, and the sequence is as shown in SEQ ID NO: 1;
   b. a breakage occurs between positions G234/D235;
   c. a breakage occurs between positions D235/E236;
   d. a breakage occurs between positions T233/ G234;
   e. a breakage occurs between positions S239/C240;
   f. a breakage occurs between positions K238/S239;
   g. a breakage occurs between positions C240/D241;
(4) a truncation variant resulting from a breakage between positions S239/C240 in one chain of etanercept and one of the following options a to g for the other chain:
   a. no breakage occurs, and the sequence is as shown in SEQ ID NO: 1;
   b. a breakage occurs between positions G234/D235;
   c. a breakage occurs between positions D235/E236;
   d. a breakage occurs between positions T233/ G234;
   e. a breakage occurs between positions S239/C240;
   f. a breakage occurs between positions K238/S239;
   g. a breakage occurs between positions C240/D241;
(5) a truncation variant resulting from a breakage between positions K238/S239 in one chain of etanercept and one of the following options a to g for the other chain:
   a. no breakage occurs, and the sequence is as shown in SEQ ID NO: 1;
   b. a breakage occurs between positions G234/D235;
   c. a breakage occurs between positions D235/E236;
   d. a breakage occurs between positions T233/ G234;
   e. a breakage occurs between positions S239/C240;
   f. a breakage occurs between positions K238/S239;
   g. a breakage occurs between positions C240/D241; and
(6) a truncation variant resulting from a breakage between positions C240/D241 in one chain of etanercept and one of the following options a to g for the other chain:
   a. no breakage occurs, and the sequence is as shown in SEQ ID NO: 1;
   b. a breakage occurs between positions G234/D235;
   c. a breakage occurs between positions D235/E236;
   d. a breakage occurs between positions T233/ G234;
   e. a breakage occurs between positions S239/C240;
   f. a breakage occurs between positions K238/S239;
   g. a breakage occurs between positions C240/D241.

The above positions are numbered with reference to SEQ ID NO: 1.

The present disclosure has one or more of the following advantageous effects.

A series of truncation variants of etanercept have been identified in the present disclosure. By analysis and identification, their specific sequences and structures have been determined. Further, methods for purifying etanercept have been optimized. It was found that the addition of sodium sulfate to an elution buffer for the protein A affinity chromatography can significantly reduce the amounts of the above-mentioned truncation variants. The purification processes described in the present disclosure are characterized in being fast, simple, scalable, suitable for large-scale production and the like.

### Terms

All publications, patents, and patent applications mentioned in this specification are incorporated herein by reference to the same extent as if each individual publication, patent, or patent application was specifically and individually indicated to be incorporated herein by reference.

Before the disclosure is described in detail below, it is to be understood that the disclosure is not limited to the specific methodologies, protocols, and reagents described herein, as these may vary. It is also to be understood that the terms used herein are for the purpose of describing particular embodiments only and are not intended to limit the scope of the present disclosure. Unless defined otherwise, all technical and scientific terms used herein have the same meanings as commonly understood by one of ordinary skill in the art to which this disclosure pertains.

Certain embodiments disclosed herein include numerical ranges, and certain aspects of the disclosure may be described in terms of ranges. Unless otherwise indicated, it is to be understood that the numerical ranges or aspects described in terms of ranges are for purposes of simplicity and convenience only, and are not to be considered as a strict limitation on the scope of the present disclosure. Accordingly, the description in terms of a range should be considered to specifically disclose all possible sub-ranges and all possible specific numerical points within that range, as if such sub-ranges and numerical points had been expressly written herein. The above principles apply equally regardless of the width of the numerical values. When a range is employed for description, the range includes endpoints of the range.

The term "protein" refers to a product obtained by fusion of an antibody fragment with another biologically active protein using genetic engineering techniques. Such a protein has multiple biological functions due to various fused proteins, and the expressed recombinant protein does not affect the antigen-binding ability of a single chain antibody or biological characteristics of the protein to which it is fused.

The term "fragment" includes at least a portion of an intact protein. As used herein, a "fragment" of a protein molecule includes an "antigen-binding fragment" of an antibody, and the term "antigen-binding fragment" refers to a polypeptide fragment of an immunoglobulin or an antibody that specifically binds to or reacts with a selected antigen or a immunogenicity-determining portion thereof, or refers to a fusion protein product further derived therefrom, e.g., a single chain antibody.

### Brief Description of the Drawings

FIG. 1 is a schematic diagram of the molecular structure of etanercept.
FIG. 2 is a non-reduced intact molecular weight analysis of LMWS (Low molecule weights) components of the etanercept molecule after a N-saccharide cleavage, a sialic acid cleavage and a O-saccharide cleavage.
FIG. 3 is a reduced intact molecular weight analysis of LMWS (Low molecule weights) components of the etanercept molecule after a N-saccharide cleavage, a sialic acid cleavage and a O-saccharide cleavage.
FIG. 4 is a schematic diagram of cleavage sites on etanercept.
FIG. 5 is a SEC-HPLC chromatogram of etanercept before and after an optimization by affinity chromatography.

### Specific Embodiments

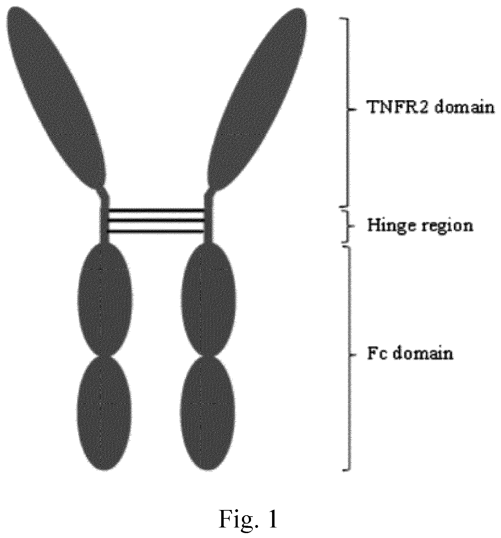

Etanercept is a fusion protein, which is a homodimer formed by two identical peptide chains linked by disulfide bonds, in particular consisting of extracellular ligand binding portions of type II tumor necrosis factor receptor and Fc fragments of human IgG1. Its structure is shown in FIG. 1. In specific embodiments, the sequence of each of the two identical peptide chains of etanercept is set forth in SEQ ID NO: 1.

In some embodiments, the truncation variants of etanercept disclosed herein comprise one or more of the following:
(1) a truncation variant resulting from a breakage between positions G234/D235 in at least one chain of etanercept,
(2) a truncation variant resulting from a breakage between positions D235/E236 in at least one chain of etanercept,
(3) a truncation variant resulting from a breakage between positions T233/G234 in at least one chain of etanercept,
(4) a truncation variant resulting from a breakage between positions S239/C240 in at least one chain of etanercept,
(5) a truncation variant resulting from a breakage between positions K238/S239 in at least one chain of etanercept, and
(6) a truncation variant resulting from a breakage between positions C240/D241 in at least one chain of etanercept,
and wherein the positions are numbered with reference to SEQ ID NO: 1.

In some specific embodiments, the truncation variants of etanercept disclosed herein mainly comprise those resulting from a breakage between positions G234/D235, and those resulting from a breakage between positions S239/C240.

In some specific embodiments, the truncation variants of etanercept disclosed herein also comprise those resulting from a breakage between positions D235/E236, T233/G234, K238/S239, or C240/D241.

In some specific embodiments, breakages between positions G234/D235, D235/E236, or T233/G234 result in loss of the receptor-binding regions (the TNFR domains that bind to TNF antigen, with the sequence L1-D235) of both heavy chains of etanercept, leading to a significant reduction in the biological activity of the resulting truncated variants.

In some specific embodiments, breakages between positions S239/C240, K238/S239 or C240/D241 result in loss of the receptor-binding region of one heavy chain, also leading to a decrease in the activity of the truncated variants.

In some specific embodiments, the truncation variants of etanercept are produced by breakages between positions G234 and D235 in both chains of etanercept, and the resulting truncation variants can be represented as HC:D235-G466 + HC:D235-G466.

In some specific embodiments, the truncation variants of etanercept are produced by a breakage between positions S239 and C240 in one chain while the other chain remains unchanged, and the resulting truncation variants can be represented as HC + HC:C240-G466.

In some specific embodiments, the truncation variants of etanercept resulting from a breakage between positions D235 and E236 in one chain and a breakage between positions G234 and D235 in the other chain can be represented as HC:E236-G466 + HC:D235-G466.

In some specific embodiments, the truncation variants of etanercept resulting from a breakage between positions T233 and G234 in one chain and a breakage between positions G234 and D235 in the other chain can be represented as HC:G234-G466 + HC:D235-G466.

In some specific embodiments, the truncation variants of etanercept are produced by a breakage between positions K238 and S239 in one chain while the other chain remains unchanged, and the resulting truncation variants can be represented as HC + HC: S239-G466.

In some specific embodiments, the truncation variants of etanercept are produced by a breakage between positions C240 and D241 in one chain while the other chain remains unchanged, and the resulting truncation variants can be represented as HC + HC:D241-G466.

The present disclosure is further described in detail by the following Examples. Specific Examples are set forth below to illustrate the disclosure. However, it is to be understood that these Examples are set forth for purposes of illustration only and are not intended to limit the scope of the disclosure.

The chromatographic media described in the present disclosure are commercially available. Cross-linked sepharose affinity chromatographic media MabSelect and MabSelect Sure used in the present disclosure were purchased from Cytiva Sweden AB.

### Example 1. Preparation of etanercept

The structure of etanercept is shown in FIG. 1. The sequence of each of the two identical peptide chains of etanercept is set forth in SEQ ID NO: 1.

### Upstream cell culture process

The host cell line used in this Example was Chinese hamster ovary CHO-K1 from China Center for Type Culture Collection. The expression vector used was an in-house expression plasmid constructed by Qilu Pharmaceutical Co., Ltd., which enabled efficient expression of exogenous recombinant proteins in mammal cells. The production medium contained the following main ingredients: glucose, sodium chloride, potassium chloride, manganese sulfate, ethanolamine, ferric citrate, zinc sulfate, copper sulfate, glutathione, magnesium chloride, sodium dihydrogen phosphate, sodium bicarbonate, alanine, asparagine, arginine, aspartic acid, cystine, cysteine, glutamic acid, glycine, histidine, isoleucine, leucine, lysine, methionine, phenylalanine, proline, serine, threonine, tryptophan, tyrosine, biotin, choline chloride, nicotinamide, vitamin B6, vitamin B2, vitamin B1, vitamin B12, and linoleic acid.

In accordance with typical cell culture processes of antibody drugs, the production of a stock solution of etanercept was carried out according to a process of cell recovery, step-by-step expansion in a shake flask and a bioreactor, and fed-batch production in a production tank. Appropriate process parameters at the production stage, such as culture temperature, pH, dissolved oxygen, stirring speed and ventilation, were set. An appropriate fed-batch culture medium and a solution of glucose were added as needed for cell growth, and a solution of an anti-foaming agent was added as needed for the culture. At the end of the culture, the cell culture was subjected to centrifugation, deep filtration and sterilization by filtration. The supernatant was collected and purified.

### Capture of etanercept protein by affinity chromatography

The MabSelect Sure affinity chromatography column (11×250mm) was equilibrated with no less than 5 column volumes of an equilibration buffer at pH 7.2 containing Tris-HCl in a concentration of 0.02mol/L and NaCl of 0.15mol/L.

The cell culture harvest solution containing the etanercept protein molecules was loaded, so that the antibody molecules were adsorbed onto the chromatographic medium. At the end of the loading, washing was performed with no less than 3 column volumes of the equilibration buffer at pH 7.2 containing Tris-HCl of 0.02mol/L and NaCl of 0.15mol/L, and then no less than 5 column volumes of Wash1 buffer at pH 7.2 containing Tris-HCl in a concentration of 0.02mol/L and sodium chloride of 1 mol/L, followed by no less than 3 column volumes of Wash2 buffer at pH 5.5 containing acetic acid-sodium acetate in a concentration of 0.02mol/L. Finally, elution was performed with an elution buffer containing acetic acid in a concentration of 0.05 mol/L at pH 3.6±0.2. The collection was performed during an interval of from the starting point at 200mAU to a point at 200mAU passing the peak. A solution of etanercept harvested by affinity chromatography was obtained. The obtained sample was detected by a SEC-HPLC detection method, and had SEC-LMWS (Low molecule weights) of 2.1%. Fragments were at a high level.

### Example 2. Characterization of protein fragments of etanercept by mass spectrometry

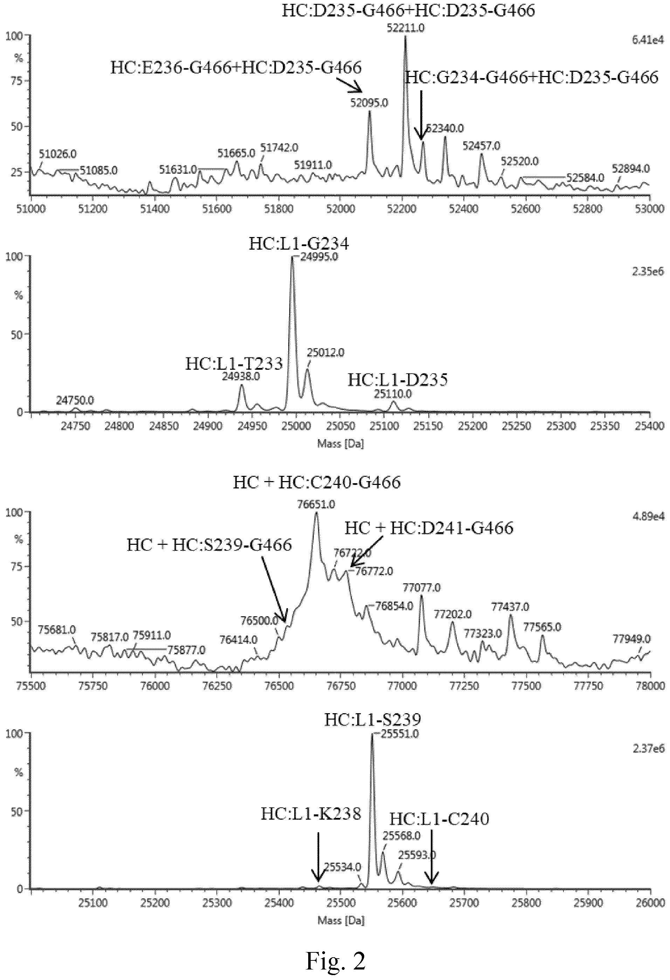

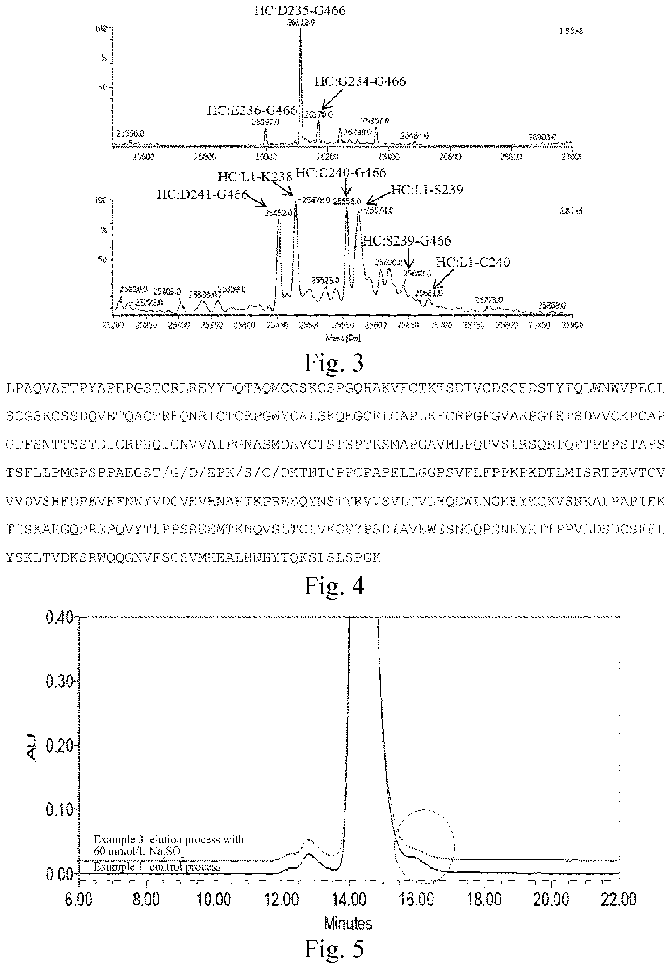

The SEC-LMWS components in the sample collected by affinity chromatography in Example 1 were separated and collected by a SEC-HPLC method. The separated and collected LMWS samples were subjected to a N-saccharide cleavage, a sialic acid cleavage and a O-saccharide cleavage, followed by non-reduced and reduced intact molecular weight analysis. Results of the non-reduced intact molecular weight analysis are shown in FIG. 2, and results of the reduced intact molecular weight analysis are shown in FIG. 3 (positions in FIG. 2 and FIG. 3 are numbered with reference to SEQ ID NO: 1). The main types of the LMWS components resulting from breakages between positions G234/D235 or S239/C240, which result in HC:D235-G466+HC:D235-G466 and HC:L1-G234 components, and HC+HC:C240-G466 and HC:L1-S239 components, respectively. In addition, a small amount of breakages were found between positions D235/E236, T233/G234, K238/S239, or C240/D241, resulting in HC:E236-G466+HC:D235-G466 and HC:L1-D235 components, HC:G234-G466+HC:D235-G466 and HC:L1-T233 components, HC+HC:S239-G466 and HC:L1-K238 components, and HC+HC:D241-G466 and HC:L1-C240 components, respectively.

The breakages for the LMWS components all occured upstream of the hinge region. The breakage sites were shown in FIG. 4. A total of six breakage sites were found in the LMWS components. Breakages at three sites G234/D235, D235/E236 and T233/G234 resulted in a loss of the receptor binding regions of both heavy chains (the sequence of the TNFR domain for binding to a TNF antigen was L1-D235), and this type of LMWS components would be inactive. Breakages at the other three sites S239/C240, K238/S239, and C240/D241 could result in a loss of the receptor binding region of one of the heavy chains, and this truncation type of LMWS components would also have a reduced activity.

Due to the loss of receptor binding regions in the series of truncation variants of etanercept mentioned above, their biological activities were necessarily be significantly affected. In order to improve the efficacy of an etanercept product, further optimization of the process is required to reduce the amounts of these variants in the etanercept product.

### Example 3. Effect of sodium sulfate used in affinity chromatography on the amount of protein fragments of etanercept

### Sample source

In accordance with Example 1, a cell harvest solution containing etanercept protein was obtained. The harvest solution was subjected to affinity chromatography, in which fragments of the etanercept were optimized with an elution buffer containing a certain concentration of sodium sulfate.

### Affinity chromatography

The MabSelect Sure affinity chromatography column (11×250mm) was equilibrated with no less than 5 column volumes of the equilibration buffer at pH 7.2 containing Tris-HCl in a concentration of 0.02mol/L and NaCl of 0.15mol/L.

The cell culture harvest solution containing etanercept protein molecules was loaded, so that the antibody molecules were adsorbed onto the chromatographic medium. At the end of the loading, washing was performed with no less than 3 column volumes of the equilibration buffer at pH 7.2 containing Tris-HCl of 0.02mol/L and NaCl of 0.15mol/L, and then no less than 5 column volumes of Wash1 buffer at pH 7.2 containing Tris-HCl in a concentration of 0.02mol/L and sodium chloride of 1 mol/L, followed by no less than 3 column volumes of Wash2 buffer at pH 5.5 containing acetic acid-sodium acetate in a concentration of 0.02mol/L. Finally, elution was performed with an elution buffer at pH 3.6±0.2 containing acetic acid in a concentration of 0.05 mol/L and sodium sulfate in various concentrations (5mmol/L, 15 mmol/L, 30 mmol/L, 45 mmol/L, and 60mmol/L) respectively. The collection was performed during an interval of from the starting point at 200mAU to a point at 200mAU passing the peak. A solution of etanercept harvested by affinity chromatography for fragment optimization was obtained. The obtained sample was detected by the SEC-HPLC detection method. As shown by the results in Table 1, SEC-LMWS (Low molecule weights) were reduced to 1.5% to 1.7%, which was 19% to 29% lower than that of the control process in Example 1.

**Table 1**

| Sodium sulfate concentration for elution | SEC-HPLC (% ) | | | |
|---|---|---|---|---|
| | High molecule weights | Monomer | Low molecule weights | Reduction compared to control process |
| 5mmol/L Na₂SO₄ | 2.7 | 95.6 | 1.7 | 19% |
| 15mmol/L Na₂SO₄ | 2.7 | 95.6 | 1.7 | 19% |
| 30mmol/L Na₂SO₄ | 2.4 | 96.0 | 1.6 | 24% |
| 45mmol/L Na₂SO₄ | 2.7 | 95.7 | 1.6 | 24% |
| 60mmol/L Na₂SO₄ | 2.7 | 95.8 | 1.5 | 29% |

The changes in SEC-LMWS (Low molecule weights) contents in the sampels before and after affinity chromatography for optimization can be seen from the SEC-HPLC chromatogram in FIG. 5.

### Example 4. Effect of sodium chloride used in affinity chromatography on the amount of protein fragments of etanercept

### Sample source

In accordance with Example 1, a cell harvest solution containing etanercept protein was obtained. The solution was subjected to affinity chromatography, in which fragments of the etanercept were optimized with an elution buffer containing a certain concentration of sodium chloride.

### Affinity chromatography

The MabSelect Sure affinity chromatography column (11×250mm) was equilibrated with no less than 5 column volumes of the equilibration buffer at pH 7.2 containing Tris-HCl in a concentration of 0.02mol/L and NaCl of 0.15mol/L.

The cell culture harvest solution containing etanercept protein molecules was loaded, so that the antibody molecules were adsorbed onto the chromatographic medium. At the end of the loading, washing was performed with no less than 3 column volumes of the equilibration buffer at pH 7.2 containing Tris-HCl of 0.02mol/L and NaCl of 0.15mol/L, and then no less than 5 column volumes of Wash1 buffer at pH 7.2 containing Tris-HCl in a concentration of 0.02mol/L and sodium chloride of 1 mol/L, followed by no less than 3 column volumes of Wash2 buffer at pH 5.5 containing acetic acid-sodium acetate in a concentration of 0.02mol/L. Finally, elution was performed with an elution buffer at pH 3.6±0.2 containing acetic acid in a concentration of 0.05 mol/L and sodium chloride in various concentrations (15 mmol/L and 60mmol/L) respectively. The collection was performed during an interval of from the starting point at 200mAU to a point at 200mAU passing the peak. A solution of etanercept harvested by affinity chromatography for fragment optimization was obtained. The obtained sample was detected by the SEC-HPLC detection method. As shown from the results in Table 2, SEC-LMWS (Low molecule weights) were reduced, with a lowest value of 1.9%, which was 10% lower than that of the control process in Example 1.

**Table 2**

| Sodium chloride concentration for elution | SEC-HPLC (%) | | | |
|---|---|---|---|---|
| | High molecule weights | Monomer | Low molecule weights | Reduction compared to control process |
| 15mmol/LNaCl | 1.6 | 96.3 | 2.1 | 0% |
| 60mmol/L NaCl | 1.6 | 96.5 | 1.9 | 10% |

From the experimental results in the above Examples 3-4, it was surprisingly found that during affinity chromatography, the addition of Na₂SO₄ to the elution buffer can significantly reduce the amounts of a series of truncation variants, while the use of another salt such as NaCl was unable to achieve such an significant effect.

## Claims

1. A composition comprising etanercept and a truncation variant thereof, the truncation variant comprising one or more of:
(1) a truncation variant resulting from a breakage between positions G234/D235 in at least one chain of etanercept,
(2) a truncation variant resulting from a breakage between positions D235/E236 in at least one chain of etanercept,
(3) a truncation variant resulting from a breakage between positions T233/G234 in at least one chain of etanercept,
(4) a truncation variant resulting from a breakage between positions S239/C240 in at least one chain of etanercept,
(5) a truncation variant resulting from a breakage between positions K238/S239 in at least one chain of etanercept, and
(6) a truncation variant resulting from a breakage between positions C240/D241 in at least one chain of etanercept,
wherein the positions are numbered with reference to SEQ ID NO: 1.

2. The composition of claim 1, wherein the truncation variant comprises one or more of:
(1) a truncation variant resulting from a breakage between positions G234/D235 in one chain of etanercept and one of the following options a to g for the other chain:
a. no breakage occurs, and the sequence is as shown in SEQ ID NO: 1;
b. a breakage occurs between positions G234/D235;
c. a breakage occurs between positions D235/E236;
d. a breakage occurs between positions T233/ G234;
e. a breakage occurs between positions S239/C240;
f. a breakage occurs between positions K238/S239;
g. a breakage occurs between positions C240/D241;
(2) a truncation variant resulting from a breakage between positions D235/E236 in one chain of etanercept and one of the following options a to g for the other chain:
a. no breakage occurs, and the sequence is as shown in SEQ ID NO: 1;
b. a breakage occurs between positions G234/D235;
c. a breakage occurs between positions D235/E236;
d. a breakage occurs between positions T233/ G234;
e. a breakage occurs between positions S239/C240;
f. a breakage occurs between positions K238/S239;
g. a breakage occurs between positions C240/D241;
(3) a truncation variant resulting from a breakage between positions T233/G234 in one chain of etanercept and one of the following options a to g for the other chain:
a. no breakage occurs, and the sequence is as shown in SEQ ID NO: 1;
b. a breakage occurs between positions G234/D235;
c. a breakage occurs between positions D235/E236;
d. a breakage occurs between positions T233/ G234;
e. a breakage occurs between positions S239/C240;
f. a breakage occurs between positions K238/S239;
g. a breakage occurs between positions C240/D241;
(4) a truncation variant resulting from a breakage between positions S239/C240 in one chain of etanercept and one of the following options a to g for the other chain:
a. no breakage occurs, and the sequence is as shown in SEQ ID NO: 1;
b. a breakage occurs between positions G234/D235;
c. a breakage occurs between positions D235/E236;
d. a breakage occurs between positions T233/ G234;
e. a breakage occurs between positions S239/C240;
f. a breakage occurs between positions K238/S239;
g. a breakage occurs between positions C240/D241;
(5) a truncation variant resulting from a breakage between positions K238/S239 in one chain of etanercept and one of the following options a to g for the other chain:
a. no breakage occurs, and the sequence is as shown in SEQ ID NO: 1;
b. a breakage occurs between positions G234/D235;
c. a breakage occurs between positions D235/E236;
d. a breakage occurs between positions T233/ G234;
e. a breakage occurs between positions S239/C240;
f. a breakage occurs between positions K238/S239;
g. a breakage occurs between positions C240/D241; and
(6) a truncation variant resulting from a breakage between positions C240/D241 in one chain of etanercept and one of the following options a to g for the other chain:
a. no breakage occurs, and the sequence is as shown in SEQ ID NO: 1;
b. a breakage occurs between positions G234/D235;
c. a breakage occurs between positions D235/E236;
d. a breakage occurs between positions T233/ G234;
e. a breakage occurs between positions S239/C240;
f. a breakage occurs between positions K238/S239;
g. a breakage occurs between positions C240/D241;
wherein the positions are numbered with reference to SEQ ID NO: 1.

3. The composition of claim 1 or 2, wherein the truncation variant is in a total amount of less than or equal to about 5% in the composition, as determined by a SEC-HPLC method.

4. The composition of claim 3, wherein the truncation variant is in a total amount of less than or equal to about 4.5%, less than or equal to about 4%, less than or equal to about 3.5%, less than or equal to about 3%, less than or equal to about 2.5%, less than or equal to about 2%, less than or equal to about 1.7%, or less than or equal to about 1.5% in the composition.

5. The composition of any one of claims 1-4, wherein the truncation variant is in a total amount of greater than or equal to about 0.01%, greater than or equal to about 0.05%, greater than or equal to about 0.1%, greater than or equal to about 0.5%, or greater than or equal to about 1% in the composition.

6. The composition of any one of claims 1-5, wherein the composition is prepared by a method comprising a step of purifying a cell culture harvest solution comprising etanercept protein molecules by protein A affinity chromatography, and wherein sodium sulfate is added to an elution buffer for the protein A affinity chromatography used in the step of purification.

7. A pharmaceutical formulation comprising the composition of any one of claims 1-6, and one or more pharmaceutically acceptable carriers.

8. A method for purifying etanercept, comprising purifying a composition containing etanercept protein molecules by protein A affinity chromatography, wherein sodium sulfate is added to an elution buffer for the protein A affinity chromatography used in the step of purification.

9. The method of claim 8, wherein the composition is a cell culture harvest solution containing etanercept protein molecules.

10. A method for reducing the amount of a truncation variant of etanercept in an etanercept-containing composition, comprising purifying a composition containing etanercept protein molecules by protein A affinity chromatography, wherein sodium sulfate is added to an elution buffer for the protein A affinity chromatography used in the step of purification, wherein the truncation variant comprises one or more of:
(1) a truncation variant resulting from a breakage between positions G234/D235 in at least one chain of etanercept,
(2) a truncation variant resulting from a breakage between positions D235/E236 in at least one chain of etanercept,
(3) a truncation variant resulting from a breakage between positions T233/G234 in at least one chain of etanercept,
(4) a truncation variant resulting from a breakage between positions S239/C240 in at least one chain of etanercept,
(5) a truncation variant resulting from a breakage between positions K238/S239 in at least one chain of etanercept, and
(6) a truncation variant resulting from a breakage between positions C240/D241 in at least one chain of etanercept,
and wherein the positions are numbered with reference to SEQ ID NO: 1.

11. The method of claim 10, wherein the composition is a cell culture harvest solution containing etanercept protein molecules.

12. The method of claim 10 or 11, wherein the truncation variant comprises one or more of:
(1) a truncation variant resulting from a breakage between positions G234/D235 in one chain of etanercept and one of the following options a to g for the other chain:
a. no breakage occurs, and the sequence is as shown in SEQ ID NO: 1;
b. a breakage occurs between positions G234/D235;
c. a breakage occurs between positions D235/E236;
d. a breakage occurs between positions T233/ G234;
e. a breakage occurs between positions S239/C240;
f. a breakage occurs between positions K238/S239;
g. a breakage occurs between positions C240/D241;
(2) a truncation variant resulting from a breakage between positions D235/E236 in one chain of etanercept and one of the following options a to g for the other chain:
a. no breakage occurs, and the sequence is as shown in SEQ ID NO: 1;
b. a breakage occurs between positions G234/D235;
c. a breakage occurs between positions D235/E236;
d. a breakage occurs between positions T233/ G234;
e. a breakage occurs between positions S239/C240;
f. a breakage occurs between positions K238/S239;
g. a breakage occurs between positions C240/D241;
(3) a truncation variant resulting from a breakage between positions T233/G234 in one chain of etanercept and one of the following options a to g for the other chain:
a. no breakage occurs, and the sequence is as shown in SEQ ID NO: 1;
b. a breakage occurs between positions G234/D235;
c. a breakage occurs between positions D235/E236;
d. a breakage occurs between positions T233/ G234;
e. a breakage occurs between positions S239/C240;
f. a breakage occurs between positions K238/S239;
g. a breakage occurs between positions C240/D241;
(4) a truncation variant resulting from a breakage between positions S239/C240 in one chain of etanercept and one of the following options a to g for the other chain:
a. no breakage occurs, and the sequence is as shown in SEQ ID NO: 1;
b. a breakage occurs between positions G234/D235;
c. a breakage occurs between positions D235/E236;
d. a breakage occurs between positions T233/ G234;
e. a breakage occurs between positions S239/C240;
f. a breakage occurs between positions K238/S239;
g. a breakage occurs between positions C240/D241;
(5) a truncation variant resulting from a breakage between positions K238/S239 in one chain of etanercept and one of the following options a to g for the other chain:
a. no breakage occurs, and the sequence is as shown in SEQ ID NO: 1;
b. a breakage occurs between positions G234/D235;
c. a breakage occurs between positions D235/E236;
d. a breakage occurs between positions T233/ G234;
e. a breakage occurs between positions S239/C240;
f. a breakage occurs between positions K238/S239;
g. a breakage occurs between positions C240/D241; and
(6) a truncation variant resulting from a breakage between positions C240/D241 in one chain of etanercept and one of the following options a to g for the other chain:
a. no breakage occurs, and the sequence is as shown in SEQ ID NO: 1;
b. a breakage occurs between positions G234/D235;
c. a breakage occurs between positions D235/E236;
d. a breakage occurs between positions T233/ G234;
e. a breakage occurs between positions S239/C240;
f. a breakage occurs between positions K238/S239;
g. a breakage occurs between positions C240/D241;
wherein the positions are numbered with reference to SEQ ID NO: 1.

## Amended claims

### Amended claims in accordance with Rule 137(2) EPC.

1. A method for purifying etanercept, comprising purifying a composition containing etanercept protein molecules by protein A affinity chromatography, wherein sodium sulfate is added to an elution buffer at pH 3.6±0.2 containing acetic acid in a concentration of 0.05 mol/L for the protein A affinity chromatography used in the step of purification, and the concentration of sodium sulfate is selected from 5mmol/L, 15 mmol/L, 30 mmol/L, 45 mmol/L and 60mmol/L.

2. The method of claim 1, wherein the composition is a cell culture harvest solution containing etanercept protein molecules.

3. A method for reducing the amount of a truncation variant of etanercept in an etanercept-containing composition, comprising purifying a composition containing etanercept protein molecules by protein A affinity chromatography, wherein sodium sulfate is added to an elution buffer at pH 3.6±0.2 containing acetic acid in a concentration of 0.05 mol/L for the protein A affinity chromatography used in the step of purification, and the concentration of sodium sulfate is selected from 5mmol/L, 15 mmol/L, 30 mmol/L, 45 mmol/L and 60mmol/L, wherein the truncation variant comprises one or more of:
(1) a truncation variant resulting from a breakage between positions G234/D235 in at least one chain of etanercept,
(2) a truncation variant resulting from a breakage between positions D235/E236 in at least one chain of etanercept,
(3) a truncation variant resulting from a breakage between positions T233/G234 in at least one chain of etanercept,
(4) a truncation variant resulting from a breakage between positions S239/C240 in at least one chain of etanercept,
(5) a truncation variant resulting from a breakage between positions K238/S239 in at least one chain of etanercept, and
(6) a truncation variant resulting from a breakage between positions C240/D241 in at least one chain of etanercept,
and wherein the positions are numbered with reference to SEQ ID NO: 1.

4. The method of claim 3, wherein the composition is a cell culture harvest solution containing etanercept protein molecules.

5. The method of claim 3 or 4, wherein the truncation variant comprises one or more of:
(1) a truncation variant resulting from a breakage between positions G234/D235 in one chain of etanercept and one of the following options a to g for the other chain:
a. no breakage occurs, and the sequence is as shown in SEQ ID NO: 1;
b. a breakage occurs between positions G234/D235;
c. a breakage occurs between positions D235/E236;
d. a breakage occurs between positions T233/ G234;
e. a breakage occurs between positions S239/C240;
f. a breakage occurs between positions K238/S239;
g. a breakage occurs between positions C240/D241;
(2) a truncation variant resulting from a breakage between positions D235/E236 in one chain of etanercept and one of the following options a to g for the other chain:
a. no breakage occurs, and the sequence is as shown in SEQ ID NO: 1;
b. a breakage occurs between positions G234/D235;
c. a breakage occurs between positions D235/E236;
d. a breakage occurs between positions T233/ G234;
e. a breakage occurs between positions S239/C240;
f. a breakage occurs between positions K238/S239;
g. a breakage occurs between positions C240/D241;
(3) a truncation variant resulting from a breakage between positions T233/G234 in one chain of etanercept and one of the following options a to g for the other chain:
a. no breakage occurs, and the sequence is as shown in SEQ ID NO: 1;
b. a breakage occurs between positions G234/D235;
c. a breakage occurs between positions D235/E236;
d. a breakage occurs between positions T233/ G234;
e. a breakage occurs between positions S239/C240;
f. a breakage occurs between positions K238/S239;
g. a breakage occurs between positions C240/D241;
(4) a truncation variant resulting from a breakage between positions S239/C240 in one chain of etanercept and one of the following options a to g for the other chain:
a. no breakage occurs, and the sequence is as shown in SEQ ID NO: 1;
b. a breakage occurs between positions G234/D235;
c. a breakage occurs between positions D235/E236;
d. a breakage occurs between positions T233/ G234;
e. a breakage occurs between positions S239/C240;
f. a breakage occurs between positions K238/S239;
g. a breakage occurs between positions C240/D241;
(5) a truncation variant resulting from a breakage between positions K238/S239 in one chain of etanercept and one of the following options a to g for the other chain:
a. no breakage occurs, and the sequence is as shown in SEQ ID NO: 1;
b. a breakage occurs between positions G234/D235;
c. a breakage occurs between positions D235/E236;
d. a breakage occurs between positions T233/ G234;
e. a breakage occurs between positions S239/C240;
f. a breakage occurs between positions K238/S239;
g. a breakage occurs between positions C240/D241; and
(6) a truncation variant resulting from a breakage between positions C240/D241 in one chain of etanercept and one of the following options a to g for the other chain:
a. no breakage occurs, and the sequence is as shown in SEQ ID NO: 1;
b. a breakage occurs between positions G234/D235;
c. a breakage occurs between positions D235/E236;
d. a breakage occurs between positions T233/ G234;
e. a breakage occurs between positions S239/C240;
f. a breakage occurs between positions K238/S239;
g. a breakage occurs between positions C240/D241;
wherein the positions are numbered with reference to SEQ ID NO: 1.
